# EUROPEAN PATENT APPLICATION

(11) **EP 4 364 653 A1**
(43) Date of publication of application: **08.05.2024**
(21) Application number: 22883757.1
(22) Date of filing: 30.08.2022
(51) Int. Cl.: A61B 5/0537, A61B 5/00, A61B 5/0533

(54) **ELECTRONIC DEVICE AND METHOD FOR MEASURING BODY IMPEDANCE**

(30) Priority: 18.10.2021 KR 20210138095
(71) Applicant: Samsung Electronics Co., Ltd., Suwon-si, Gyeonggi-do 16677 (KR)
(72) Inventor: CHANG, Namseok, Suwon-si, Gyeonggi-do 16677 (KR); PARK, Jaehyuck, Suwon-si, Gyeonggi-do 16677 (KR)
(74) Representative: Gulde & Partner
(86) International application number: PCT/KR2022/012975
(87) International publication number: WO 2023/068532

(57) **Abstract**

An electronic device according to various embodiments comprises: a plurality of electrodes; a sensor operatively connected to the plurality of electrodes; a memory; and a processor operatively connected to the sensor and the memory, wherein the processor may: obtain, through the sensor, a plurality of contact impedances on the basis of contact between a user and the plurality of electrodes; perform body impedance measurement when all of the obtained contact impedances are less than a first impedance value; and determine not to perform body impedance measurement when at least one of the obtained contact impedances is equal to or greater than the first impedance value. In addition, various other embodiments are possible.

## Description

### [Technical Field]

This document relates to an electronic device, for example, to a method for measuring body impedance with the electronic device.

### [Background Art]

Recently, with the advancement of mobile communication technology and processor technology, wearable devices capable of performing various functions have been developed. The electronic device in the form of a wearable device may be, for example, in the form of a wrist watch (smartwatch) attached to the user's wrist, in which case the electronic device may support not only an intrinsic clock function but also various other functions, such as sending and receiving calls and messages, executing various applications, and playing back multimedia content.

Since a wearable device operates while being attached to the user's body, it may be used to obtain user's body data through various biometric sensors. For example, the user's heart rate, stress, or blood oxygen saturation (SpO₂) can be measured, and an analysis (e.g., bioelectrical impedance analysis (BIA)) can be performed based on the measured body data. The wearable device may measure body impedance through contact between the user's body and electrodes.

### [Disclosure of Invention]

### [Technical Problem]

If the measurement is started and ended at a fixed time, as the contact time between the skin and the electrode may be not sufficient, there is a possibility that the measurement of the body impedance may become inaccurate. Because of this, it may be very difficult to fix the required time until the fluctuation range of the body impedance is reduced. To solve this problem, the electronic device according to a comparative example may utilize variations in body impedance over time in order to stably measure the body impedance. In case that the fluctuation range of the obtained body impedance is small, it may be considered that an accurate impedance value has been obtained, and the measurement can be ended.

Also, as the electronic device according to a comparative example does not provide a separate guide for measurement, it may cause inconvenience to the user. For example, if the user's skin is dry, it may be difficult to perform measurement, but the electronic device according to a comparative example may guide repeated measurement without a separate guidance when the maximum measurement time has elapsed, so there may be inconvenience in measurement.

The object of various embodiments of this document is to, in case that the electronic device measures body impedance as described above, improve accuracy by measuring body impedance under effective contact conditions, and resolve user inconvenience by providing a user-friendly interface.

### [Solution to Problem]

An electronic device according to various embodiments may include: a plurality of electrodes; a sensor module operably connected to the plural electrodes; a memory; and a processor operably connected to the sensor module and the memory, wherein the processor may be configured to: obtain plural contact impedances through the sensor module based on contact between the plural electrodes and a user; perform body impedance measurement in case that all the obtained contact impedances are less than a first impedance value; and determine not to perform body impedance measurement in case that at least one of the obtained contact impedances is greater than or equal to the first impedance value.

A method for an electronic device to measure body impedance according to various embodiments may include: obtaining, through a sensor module, at least one contact impedance based on contact between one of plural electrodes and a user; performing body impedance measurement in case that all the obtained contact impedances are less than a first impedance value; and determining not to perform body impedance measurement in case that at least one of the obtained contact impedances is greater than or equal to the first impedance value.

### [Advantageous Effects of Invention]

According to various embodiments, the electronic device may measure multiple contact impedances to improve the accuracy of body impedance measurement. For example, the electronic device may determine whether to measure the body impedance based on a contact impedance value, and measure an effective measurement time taken for the measurement. The electronic device may end the measurement based on the effective measurement time, the contact impedance value, and the fluctuation range of the body impedance value. Thereby, the electronic device may flexibly control the time taken to measure the body impedance.

According to various embodiments, the electronic device may adjust various threshold values based on the user's physical characteristics. For example, as it may be difficult to measure body impedance of a user with relatively dry skin, the first impedance value, which is a measurement start threshold, may be increased. The electronic device may provide a visual interface and a voice interface to guide the user of the progress of impedance measurement.

Other effects obtainable or predicted by various embodiments of the electronic device will be explicitly or implicitly disclosed in the detailed description of the embodiments of the electronic device. For example, various effects predicted according to various embodiments of the electronic device will be disclosed in the following detailed description.

### [Brief Description of Drawings]

FIG. 1 is a block diagram of an electronic device in a network environment according to various embodiments.
FIG. 2 is a front perspective view of an electronic device according to various embodiments disclosed in this document.
FIG. 3 is a rear perspective view of the electronic device in FIG. 2.
FIG. 4 is an exploded perspective view of the electronic device in FIG. 2.
FIG. 5 is a block diagram of an electronic device according to various embodiments.
FIGS. 6A, 6B and 6C are graphs depicting contact impedance values obtained by the electronic device over time according to various embodiments.
FIGS. 7A and 7B illustrate an example in which an exclusion time occurs during body impedance measurement of the electronic device according to various embodiments.
FIGS. 8A, 8B and 8C illustrate an example in which body impedance measurement of the electronic device is ended according to various embodiments.
FIGS. 9A and 9B illustrate a visual interface provided by the electronic device to the user according to various embodiments.
FIG. 10 is a flowchart depicting a method for the electronic device to measure body impedance according to various embodiments.
FIG. 11 is a flowchart depicting a method for the electronic device to start measurement according to various embodiments.
FIG. 12 is a flowchart illustrating a case in which an exclusion time occurs during measurement of the electronic device according to various embodiments.
FIG. 13 is a flowchart for the electronic device to end measurement according to various embodiments.

### [Mode for the Invention]

Hereinafter, embodiments of the disclosure will be described in detail with reference to the accompanying drawings.

In the description of the embodiments, descriptions of technical contents that are well known in the art to which the disclosure pertains and are not directly related to the disclosure will be omitted. In addition, detailed descriptions of components having substantially the same structure and function will be omitted.

Likewise, some components are exaggerated, omitted, or schematically depicted in the accompanying drawings, and the size of each component does not necessarily reflect the actual size. Accordingly, the disclosure is not limited to or by the relative sizes or spacings drawn in the accompanying drawings.

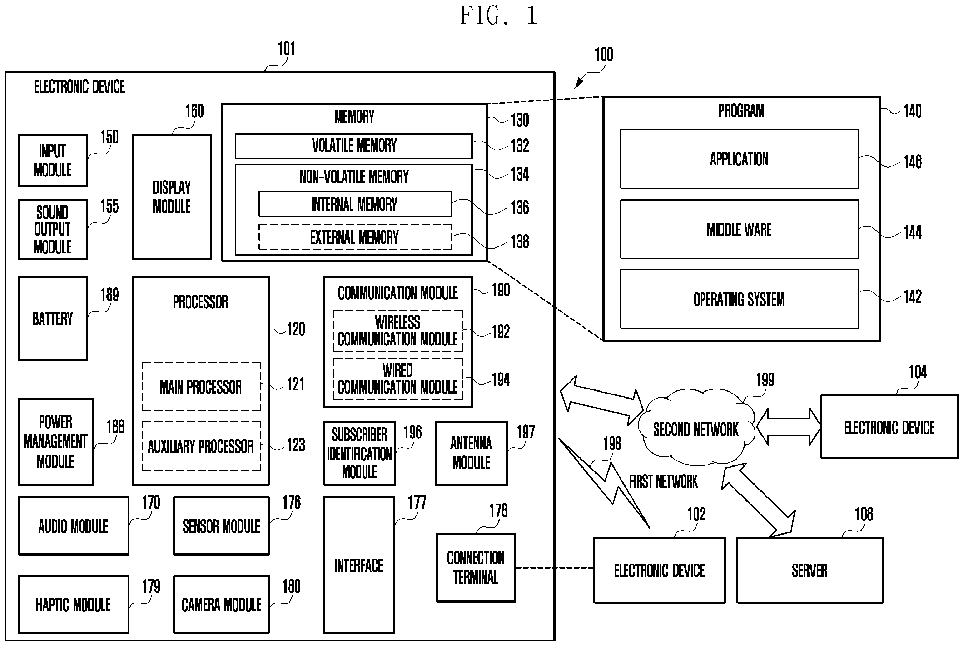

Fig. 1 is a block diagram illustrating an electronic device 101 in a network environment 100 according to various embodiments.

Referring to Fig. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thererto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as BluetoothTM, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adj acent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStoreTM), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

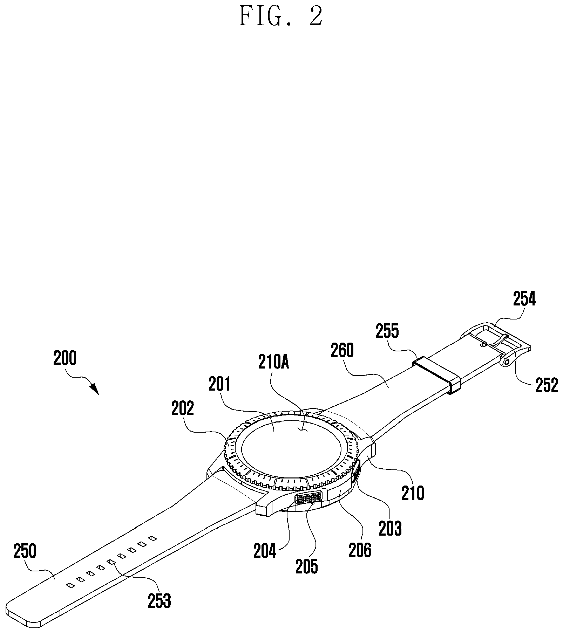

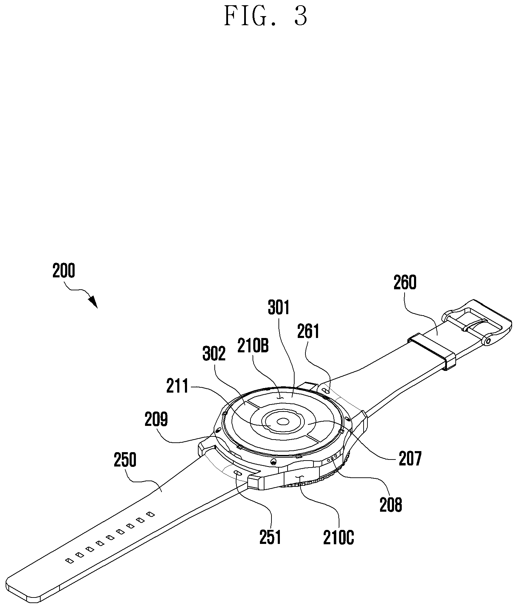

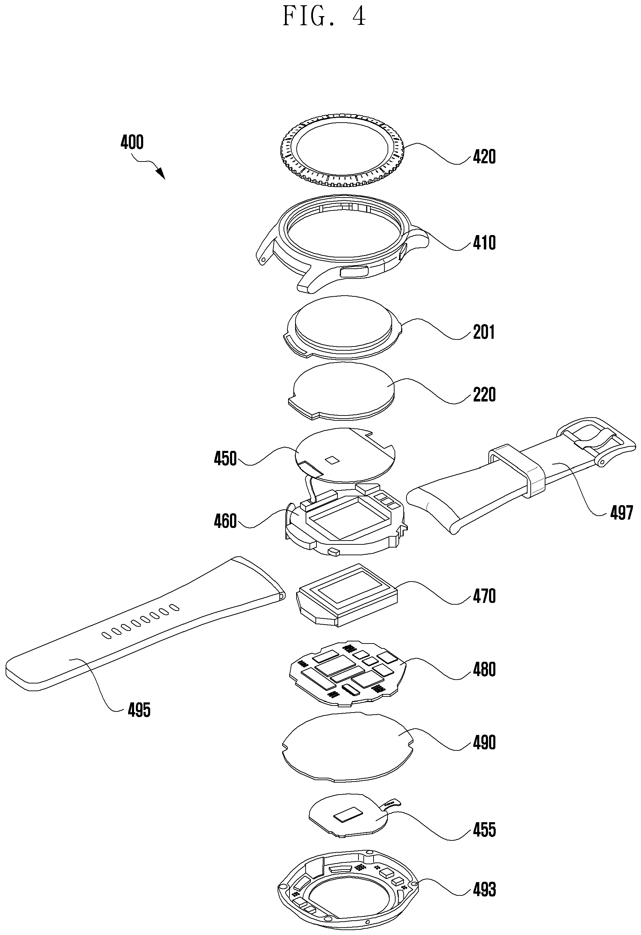

FIG. 2 is a front perspective view of an electronic device according to various embodiments disclosed in this document. FIG. 3 is a rear perspective view of the electronic device in FIG. 2. FIG. 4 is an exploded perspective view of the electronic device in FIG. 2.

The electronic device 200 shown in FIGS. 2 to 4 may be an instance of the electronic device 101 described with reference to FIG. 1. Hence, although not mentioned below, the electronic device 200 may include the components described in FIG. 1.

With reference to FIGS. 2 and 3, the electronic device 200 according to an embodiment may include: a housing 210 including a first surface (or, front surface) 210A, a second surface (or, rear surface) 210B, and a side surface 210C surrounding the space between the first surface 210A and the second surface 210B; and fastening members 250 and 260 connected to at least a portion of the housing 210 and configured to detachably fasten the electronic device 200 to a body part (e.g., wrist, ankle) of the user. In another embodiment (not shown), the housing may refer to a structure forming some of the first surface 210A, the second surface 210B, and the side surface 210C in FIGS. 2 and 3. According to an embodiment, the first surface 210A may be formed by a front plate 201 that is substantially transparent at least in part (e.g., glass plate containing various coating layers, or polymer plate). The second surface 210B may be formed by a rear plate 207 that is substantially opaque. The rear plate 207 may be made of, for example, coated or colored glass, ceramic, polymer, metal (e.g., aluminum, stainless steel (STS), or magnesium), or a combination thereof. The side surface 210C may be formed by a side bezel structure (or, side member) 206 that is coupled to the front plate 201 and the rear plate 207 and contains a metal and/or a polymer.

In a certain embodiment, the rear plate 207 and the side bezel structure 206 may be integrally formed and contain the same material (e.g., metal material such as aluminum). The fastening members 250 and 260 may be made of various materials and formed in various shapes. For example, the fastening members 250 and 260 may be formed as a single body or as plural unit links that are movable with each other, by woven material, leather, rubber, urethane, metal, ceramic, or a combination thereof.

According to an embodiment, the electronic device 200 may include at least one of a display 220 (refer to FIG. 4), an audio module (205, 208), a sensor module 211, key input devices 202, 203 and 204, or a connector hole 209. In a certain embodiment, at least one of the components (e.g., key input device 202, 203 or 204, connector hole 209, or sensor module 211) may be removed from the electronic device 200, or a different component may be added to the electronic device 200.

In one embodiment, the display 220 can be visually exposed through a significant portion of the front plate 201. The display 220 may have a shape corresponding to the shape of the front plate 201 and may be in various shapes such as a circle, an ellipse, and a polygon. The display 220 may be disposed in combination with or adj acent to a touch sensing circuit, a pressure sensor capable of measuring the intensity (pressure) of a touch, and/or a fingerprint sensor.

In one embodiment, the audio module (205, 208) may include a microphone hole 205 and a speaker hole 208. In the microphone hole 205, a microphone for picking up external sounds may be disposed therein, and plural microphones may be arranged to sense the direction of a sound in a certain embodiment. The speaker hole 208 can be used for an external speaker and a call receiver. In a certain embodiment, the speaker hole 208 and the microphone hole 203 may be implemented as a single hole, or a speaker may be included without the speaker hole 208 (e.g., piezo speaker).

In one embodiment, the sensor module 211 may generate an electrical signal or data value corresponding to an internal operating state of the electronic device 200 or an external environmental state. The sensor module 211 may include, for example, a biometric sensor module 211 (e.g., HRM sensor) disposed on the second surface 210B of the housing 210. The electronic device 200 may further include a sensor module (not shown) including at least one of, for example, a gesture sensor, a gyro sensor, an air pressure sensor, a magnetic sensor, an acceleration sensor, a grip sensor, a color sensor, an infrared (IR) sensor, a biometric sensor, a temperature sensor, a humidity sensor, or an illuminance sensor.

In one embodiment, the sensor module 211 may include electrodes (or electrode regions) 301 and 302 that constitute a part of the surface of the electronic device 200 and a biometric signal detection circuit (not shown) electrically connected to the electrodes 301 and 302. For example, the electrodes 301 and 302 may include a first electrode 301 and second electrode 302 disposed on the second surface 210B of the housing 210. The sensor module 211 may be configured such that the electrodes 301 and 302 obtain an electrical signal from a body part of the user, and the biometric signal detection circuit detects biometric information of the user based on the electrical signal. For example, the biometric information may be heart rate information or atrial fibrillation information based on an electrocardiogram (ECG). As another example, the biometric information may be body composition information, body fat information, or body water information based on bioelectrical impedance analysis (BIA). As another example, the biometric information may be skin moisture level information based on a galvanic skin response (GSR).

In one embodiment, the electronic device 200 may include plural electrodes that can come into contact with the user's body. The plural electrodes may include, for example, the electrodes 301 and 302 disposed on the second surface 210B of the electronic device as shown in FIG. 3, and an electrode (not shown) disposed on the first surface 210A and/or the side surface 210C of the electronic device. The plural electrodes may be connected to each other in a circuit, but portions functioning as electrodes may be segmented from each other. For example, the electrodes may be composed of three electrodes including the electrodes 301 and 302 disposed on the second surface 210B and the electrode disposed on the side surface 210C. Various biometric information of the user may be detected through the plural electrodes. In one embodiment, information related to a user's electrocardiogram may be measured by using the plural electrodes. ECG measurement can be performed in a variety of ways. For example, in ECG measurement, the plural electrodes described above may include an INP (positive) electrode (e.g., electrode 301), an INM (negative) electrode, and a RLD (right-leg drive) electrode (e.g., electrode 302). ECG measurement can be performed through the INP electrode and RLD electrode. Here, the RLD electrode may be a connection point used to increase the ECG measurement performance by reducing a signal having the same phase at an electrode in contact with the body.

In one embodiment, the key input devices 202, 203 and 204 may include a wheel key 202 disposed on the first surface 210A of the housing 210 and rotatable in at least one direction, and/or side key buttons 203 and 204 disposed on the side surface 210C of the housing 210. The wheel key 202 may have a shape corresponding to the shape of the front plate 201. In another embodiment, the electronic device 200 may not include some or all of the key input devices 202, 203 and 204 described above, and the key input device 202, 203 or 204 that is not included may be implemented in other forms, such as soft keys, on the display 220. The connector hole 209 may accommodate a connector (e.g., USB connector) for transmitting and receiving power and/or data to and from an external electronic device, and may include another connector hole (not shown) that can accommodate a connector for transmitting and receiving an audio signal to and from an external electronic device. The electronic device 200 may further include, for example, a connector cover (not shown) that covers at least a portion of the connector hole 209 and blocks foreign substances from entering the connector hole.

In one embodiment, the fastening members 250 and 260 may be detachably fastened to at least a portion of the housing 210 by using locking members 251 and 261. The fastening members 250 and 260 may include one or more of a fixing member 252, fixing member fastening holes 253, a band guide member 254, and a band fixing ring 255.

In one embodiment, the fixing member 252 may be formed to fix the housing 210 and the fastening members 250 and 260 to a body part (e.g., wrist, ankle) of the user. The fixing member fastening holes 253 may fix the housing 210 and the fastening members 250 and 260 to a body part of the user in correspondence to the fixing member 252. The band guide member 254 may be formed to limit the range of movement of the fixing member 252 when the fixing member 252 engages with a fixing member fastening hole 253, so that the fastening members 250 and 260 may be fastened in close contact to a body part of the user. The band fixing ring 255 may limit the range of movement of the fastening members 250 and 260 while the fixing member 252 and the fixing member fastening hole 253 are fastened.

With reference to FIG. 4, the electronic device 400 may include a side bezel structure 410, a wheel key 420, a front plate 201, a display 220, a first antenna 450, a second antenna 455, a support member 460 (e.g., bracket), a battery 470, a printed circuit board 480, a sealing member 490, a rear plate 493, and fastening members 495 and 497. At least one of the components of the electronic device 400 may be the same as or similar to at least one of the components of the electronic device 200 of FIG. 2 or 3, and repeated descriptions will be omitted below. The support member 460 may be disposed inside the electronic device 400 and connected to the side bezel structure 410, or may be integrally formed with the side bezel structure 410. The support member 460 may be made of, for example, a metal material and/or a non-metal (e.g., polymer) material. The display 220 may be coupled to one surface of the support member 460 and the printed circuit board 480 may be coupled to the other surface. A processor, a memory, and/or an interface may be mounted on the printed circuit board 480. The processor may include, for example, one or more of a central processing unit, an application processor, a graphics processing unit, an application processor signal processing unit, or a communication processor.

The memory may include, for example, a volatile memory or a non-volatile memory. The interface may include, for example, a high definition multimedia interface (HDMI), a universal serial bus (USB) interface, an SD card interface, and/or an audio interface. The interface may electrically or physically connect, for example, the electronic device 400 to an external electronic device, and may include a USB connector, an SD card/MMC connector, or an audio connector.

The battery 470 may supply power to at least one component of the electronic device 400, and may include, for example, a primary cell which is not rechargeable, a secondary cell which is rechargeable, or a fuel cell. At least a portion of the battery 470 may be disposed substantially coplanar with, for example, the printed circuit board 480. The battery 470 may be integrally disposed inside the electronic device 200, or may be disposed detachably from the electronic device 200.

The first antenna 450 may be disposed between the display 220 and the support member 460. The first antenna 450 may include, for example, a near field communication (NFC) antenna, a wireless charging antenna, and/or a magnetic secure transmission (MST) antenna. The first antenna 450 may perform short-range communication with, for example, an external device or wirelessly transmit or receive power required for charging, and may transmit a short-range communication signal or a magnetic-based signal containing payment data. In another embodiment, an antenna structure may be formed by a part of the side bezel structure 410 and/or the support member 460 or a combination thereof.

The second antenna 455 may be disposed between the printed circuit board 480 and the rear plate 493. The second antenna 455 may include, for example, a near field communication (NFC) antenna, a wireless charging antenna, and/or a magnetic secure transmission (MST) antenna. The second antenna 455 may perform short-range communication with, for example, an external device or wirelessly transmit or receive power required for charging, and may transmit a short-range communication signal or a magnetic-based signal containing payment data. In another embodiment, an antenna structure may be formed by a part of the side bezel structure 410 and/or the rear plate 493 or a combination thereof.

The sealing member 490 may be positioned between the side bezel structure 410 and the rear plate 493. The sealing member 490 may be formed to block moisture and foreign substances from flowing into a space surrounded by the side bezel structure 410 and the rear plate 493 from the outside.

According to various embodiments, the electronic device disclosed in this document is not limited to the form illustrated in FIGS. 2 to 4. For example, the electronic device may include a wearable electronic device in a form that can be worn by a user, such as in the form of a smart ring or smart glasses.

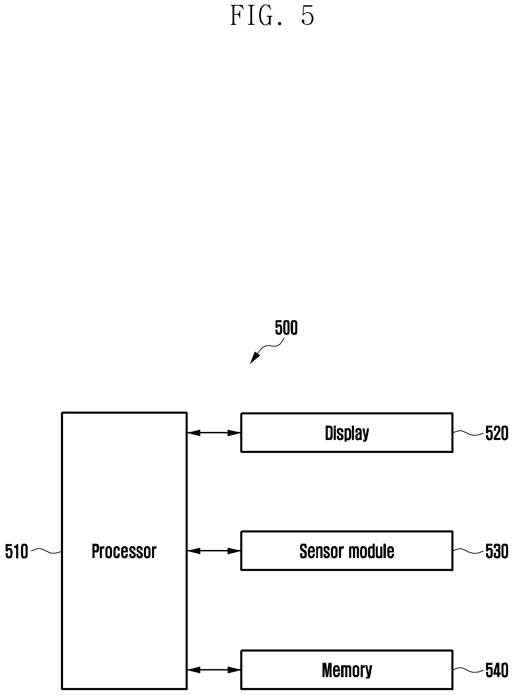

FIG. 5 is a block diagram of an electronic device according to various embodiments.

With reference to FIG. 5, the electronic device 500 may include a display 520, a sensor module 530, a processor 510, and a memory 540, and some of the illustrated components may be omitted or replaced in various embodiments. The electronic device 500 may further include at least some of the configurations and/or functions of the electronic device 101 in FIG. 1. At least some of the illustrated (or not illustrated) components of the electronic device 500 may be operably, functionally, and/or electrically connected.

According to various embodiments, the display 520 may display various images under the control of the processor 510. The display 520 may be implemented with, but not limited to, any one of liquid crystal display (LCD), light-emitting diode (LED) display, micro LED display, quantum dot (QD) display, or organic light-emitting diode (OLED) display. The display 520 may be formed of a touchscreen that senses a touch and/or proximity touch (or hovering) input using a user's body part (e.g., finger) or an input device (e.g., stylus pen). The display 520 may include at least some of the configuration and/or function of the display module 160 in FIG. 1.

According to various embodiments, at least a portion of the display 520 may be flexible, and may be implemented with a foldable display or a rollable display.

According to various embodiments, the sensor module 530 (e.g., sensor module 176 in FIG. 1) may sense an operating state (e.g., power or temperature) of the electronic device 101 or an external environmental state (e.g., user state), and may generate an electrical signal or data value corresponding to the sensed state. According to an embodiment, the sensor module 530 may include, for example, a gesture sensor, a gyro sensor, a barometric pressure sensor, a magnetic sensor, an acceleration sensor, a grip sensor, a proximity sensor, a color sensor, an IR (infrared) sensor, a biometric sensor, a temperature sensor, a humidity sensor, or an illuminance sensor.

According to an embodiment, the sensor module 530 may sense at least one biometric signal. For example, the sensor module 530 may measure contact impedance between the user's body and the electrode, and may measure body impedance by emitting light into the user's body and receiving the reflected light. According to an embodiment, the sensor module 530 may include a plurality of electrodes (e.g., four or more), and contact impedance may be generated at each electrode contacting the body. The sensor module 530 may measure the contact impedance generated at each electrode. According to an embodiment, the individual electrodes of the sensor module 530 may contact different body parts. For example, the sensor module 530 may include a first electrode, a second electrode, a third electrode, and a fourth electrode. The first electrode and the second electrode may contact a user's finger, and the third electrode and the fourth electrode may contact a user's wrist. The sensor module 530 may measure a first contact impedance generated by the first electrode contacting the finger, a second contact impedance generated by the second electrode contacting the finger, a third contact impedance generated by the third electrode contacting the wrist, and a fourth contact impedance generated by the fourth electrode contacting the wrist. Hereinafter, the sensor module 530 will be described as having four electrodes, but the sensor module 530 may include a variable number of electrodes, and each electrode may come into contact with a different body part to generate contact impedance, which is not limited to the example described above.

According to various embodiments, the memory 540 may include a volatile memory (e.g., volatile memory 132 in FIG. 1) and a non-volatile memory (e.g., non-volatile memory 134 in FIG. 1), and may temporarily or permanently store various data. The memory 540 may include at least some of the configurations and/or functions of the memory 130 in FIG. 1, and may store the programs 140 in FIG. 1.

According to various embodiments, the memory 540 may store various instructions that can be executed by the processor 510. These instructions may include arithmetic and logical operations and control commands such as data movement and input/output, which can be recognized by the processor 510.

According to various embodiments, the processor 510 may be operably, functionally, and/or electrically connected to the individual components (e.g., display 520, sensor module 530, memory 540) of the electronic device 500, and may be a configuration capable of performing operations or data processing related to control and/or communication of each component. The processor 510 may include at least some of the configurations and/or functions of the processor 120 in FIG. 1.

According to various embodiments, although there will be no limitations on the operations and data processing functions that the processor 510 can implement on the electronic device 500, various embodiments for measuring body impedance by using the electronic device 500 will be described below. Operations of the processor 510 to be described later may be carried out by loading instructions stored in the memory 540.

According to various embodiments, the processor 510 may determine the threshold value based on the body characteristics of the user. The processor 510 may obtain various body characteristics of the user by emitting light toward the human body through the sensor module 530 and receiving the reflected light reflected from internal structures of the human body, such as blood vessels, muscles, and bones. For example, the processor 510 may obtain body characteristics of the user such as temperature, humidity, heart rate, blood oxygen saturation (SpO₂), stress index, and blood flow velocity through the sensor module 530, and store them in the memory 540. The processor 510 may determine a threshold value required for measuring body impedance based on the obtained body characteristics of the user. For example, the processor 510 may determine various thresholds, such as a first impedance value being a measurement start threshold, a second impedance value being a measurement end threshold, a first time being a measurement time threshold, a second time being an exclusion time threshold, and a fluctuation threshold. The contact impedance is an impedance generated between the electrode of the electronic device 500 and the user's body, and may interfere with measurement of the body impedance. The processor 510 may set the first impedance value, which is the upper limit of the contact impedance, in consideration of the accuracy of body impedance measurement. For example, when the user's skin is dry, it may be relatively difficult for the sensor module 530 to measure the body impedance. When a user is determined to have dry skin as a result of measuring the user's skin humidity, the processor 510 may determine the first impedance value of the user to be higher than the first impedance value of a user having relatively less dry skin.

According to various embodiments, the processor 510 may obtain at least one contact impedance through the sensor module 530. According to an embodiment, the processor 510 may determine the maximum value of the obtained at least one contact impedance. For example, the processor 510 may obtain a first contact impedance, a second contact impedance, a third contact impedance, and a fourth contact impedance through the sensor module 530. The processor 510 may compare the first contact impedance, the second contact impedance, the third contact impedance, and the fourth contact impedance, and determine the largest value as the maximum value. According to an embodiment, the processor 510 may obtain at least one contact impedance value through the sensor module 530 at a preset time interval. Whenever it receives as many contact impedance values as the number of electrodes, the processor 510 may determine the maximum value of the received values. The electrode having the maximum value may be changed according to the state of the user's body currently in contact and the contact impedance value measured by the sensor module 530. For example, the processor 510 may determine the first contact impedance as the maximum value at a first time point, and determine the second contact impedance as the maximum value at a second time point. According to an embodiment, the processor may utilize the determined maximum value when comparing measured values with the various thresholds.

According to various embodiments, the processor 510 may determine the first impedance value based on the user's body state, and may compare the obtained at least one contact impedance with the first impedance value. When all the obtained contact impedances are smaller than the first impedance value, the processor 510 may measure the body impedance. The contact impedance between the electrode and the body may decrease over time. For example, when the first electrode and a user's finger make first contact, the first contact impedance of the first electrode may be greater than the first impedance value, but the first contact impedance may decrease to less than the first impedance value as time passes. In the same way, when the second contact impedance, the third contact impedance, and the fourth contact impedance decrease to less than the first impedance value, the processor 510 may measure the body impedance. According to an embodiment, the third and fourth contact impedances at the third and fourth electrodes in contact with the wrist may be lower than the first impedance value from the start of the measurement. This is because, when the user is wearing the wearable device on the wrist, the wrist and the wearable device can be in stable contact. Conversely, when at least one of the obtained contact impedances is greater than the first impedance value, the processor 510 may stop measuring the body impedance. For example, at least one of the first and second contact impedances measured at the first and second electrodes in contact with the finger may be greater than the first impedance value. When body impedance measurement is stopped, the processor 510 may stop measuring the effective measurement time and measure the exclusion time. The exclusion time may be a time during which the processor 510 does not measure the body impedance. According to an embodiment, if all contact impedances decrease to less than the first impedance value while the processor 510 measures the exclusion time, the exclusion time may be initialized. The processor 510 does not cumulatively measure the exclusion time, but may measure the exclusion time from the time when body impedance measurement is stopped.

According to various embodiments, the processor 510 may measure the effective measurement time while body impedance measurement is in progress. According to an embodiment, the processor 510 may measure the effective measurement time based on the size of the area of the electrode of the electronic device 500. According to an embodiment, the processor 510 may measure the effective measurement time further based on the effective contact area of the user's body (e.g., finger), the contact pressure, and the body moisture level. The processor 510 may obtain an effective contact area and contact pressure of the body through the sensor module 530. For example, the processor 510 may measure the effective measurement time when the effective contact area of the user's body is greater than or equal to a preset area and the contact pressure is greater than or equal to a preset pressure, and may stop measuring the effective measurement time when the effective contact area is less than the preset area or the contact pressure is less than the preset pressure. The criteria for the processor 510 to measure the effective measurement time is not limited to the above-mentioned embodiment, and an embodiment in which the processor 510 measures the effective measurement time based on the size of the area of the electrode of the electronic device 500 will be described below. According to an embodiment, when the measurement is stopped in the electronic device 500 having an electrode area less than a preset size, the processor 510 may initialize the effective measurement time. For example, if the body impedance measurement in the electronic device 500 having a small electrode area is performed for a first time and then stopped, the processor 510 may initialize the effective measurement time again to 0 seconds. According to an embodiment, when the measurement is stopped in the electronic device 500 having an electrode area greater than or equal to a preset size, the processor 510 may stop measuring the effective measurement time, and thereafter when the body impedance measurement is resumed, the processor 510 may measure the effective measurement time in a cumulative manner. For example, if body impedance measurement is performed for a first time and then stopped in the electronic device 500 having a large electrode area, the processor 510 may maintain the effective measurement time to be the first time. Thereafter, when all the contact impedances decrease again to less than the first impedance value, the processor 510 may measure the effective measurement time in a cumulative manner from the first time.

According to various embodiments, the processor 510 may not immediately determine whether to initialize or maintain the effective measurement time at the point in time when the measurement is stopped according to a given electrode area, but may intelligently determine whether to initialize or maintain the effective measurement time. When the measurement is stopped in the electronic device 500, the processor 510 may maintain the effective measurement time without initializing it regardless of the electrode area. When the condition that all the contact impedances are less than the first impedance value is satisfied again, the processor 510 may compare the individual contact impedances with an auxiliary impedance value while resuming the measurement. Here, the auxiliary impedance value may be determined to be a value smaller than the first impedance value and larger than the second impedance value to be described later. If all the contact impedances are less than the auxiliary impedance value, the processor 510 may maintain the effective measurement time. On the other hand, if at least one contact impedance is greater than the auxiliary impedance value and less than the first impedance value, the processor 510 may initialize the effective measurement time.

According to various embodiments, the processor 510 may end the body impedance measurement based on at least one of the measured values. Next, an embodiment in which the processor 510 ends the body impedance measurement will be described. According to an embodiment, the processor 510 may end the measurement when all the contact impedances are less than the second impedance value. When all the contact impedances are less than the second impedance value, the processor 510 may determine that the contact impedance is low enough not to interfere with the body impedance measurement. According to an embodiment, the processor 510 may end the measurement when all contact impedances obtained for a preset time are less than the second impedance value.

According to an embodiment, the processor 510 may end the body impedance measurement in case that the range of variation of the measured body impedance is less than a preset range. The processor 510 may measure the fluctuation range of the obtained body impedance and determine whether the fluctuation range is less than the preset range. For example, the processor 510 may identify a maximum value and a minimum value among body impedances obtained for a preset time, and calculate a difference between the maximum value and the minimum value. When the difference between the maximum value and the minimum value calculated by the processor 510 is less than the preset range, the processor 510 may determine that the body impedance has been stably measured and end the measurement. Conversely, in case that the difference between the maximum value and the minimum value calculated by the processor 510 is greater than or equal to the preset range, the processor 510 may determine that the accuracy of the body impedance measurement is not sufficient and may continue the measurement.

According to an embodiment, the processor 510 may end the measurement when the effective measurement time is longer than or equal to the first time (e.g., 30 seconds). When the effective measurement time is longer than or equal to the first time, the processor 510 may determine that the body impedance measurement has been sufficiently performed and may end the measurement. For example, although at least one of the obtained contact impedances is greater than or equal to the second impedance value, and the body impedance variation value is greater than or equal to the preset range, if the effective measurement time is longer than or equal to the first time, the processor 510 may end the measurement.

According to an embodiment, the processor 510 may end the measurement in case that the exclusion time is longer than or equal to a second time. Unlike the above-mentioned three embodiments, in case that the exclusion time is longer than or equal to the threshold value, the processor 510 may treat it as a measurement failure. For example, when at least one of the obtained contact impedances is greater than or equal to the first impedance value, the processor 510 may start to measure the exclusion time. When the duration in which at least one of the obtained contact impedances is greater than or equal to the first impedance value continues for the second time or longer, the processor 510 may determine that the body impedance measurement is a measurement failure and end the measurement. Similarly, although the measurement is stopped because the contact impedance increases to more than the first impedance value while performing the body impedance measurement, if the exclusion time is longer than or equal to the threshold value, the processor 510 may end the measurement. According to various embodiments, the processor 510 may end the body impedance measurement when any one of the aforementioned conditions is satisfied.

According to various embodiments, after the body impedance measurement is ended, the processor 510 may obtain at least one body composition data based on the obtained body impedance. For example, the processor 510 may obtain body composition data such as body fat, skeletal muscles, and body water.

According to various embodiments, the processor 510 may guide a measurement situation to the user by using various interfaces. According to an embodiment, the processor 510 may provide a visual interface including a graphical object and text indicating the effective measurement time and the exclusion time. For example, the processor 510 may provide an interface in which the effective measurement time, the exclusion time, and the end time are represented in different colors to thereby guide the user of the progress of the body impedance measurement. According to an embodiment, the processor 510 may guide the measurement situation through a voice interface. For example, the processor 510 may notify, through a voice message, the user of whether the measurement is currently in progress, or whether the measurement is stopped because at least one of obtained contact impedances is greater than or equal to the first impedance value.

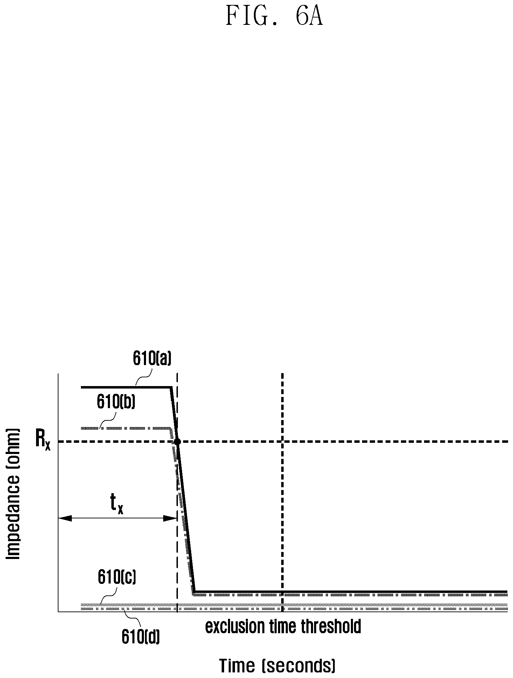

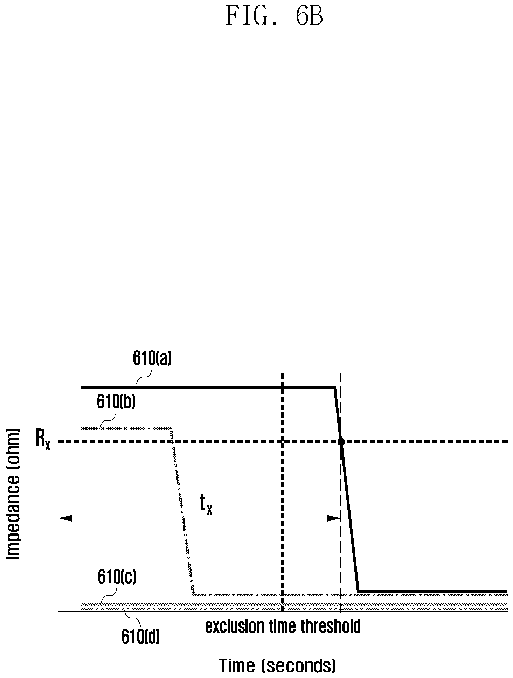

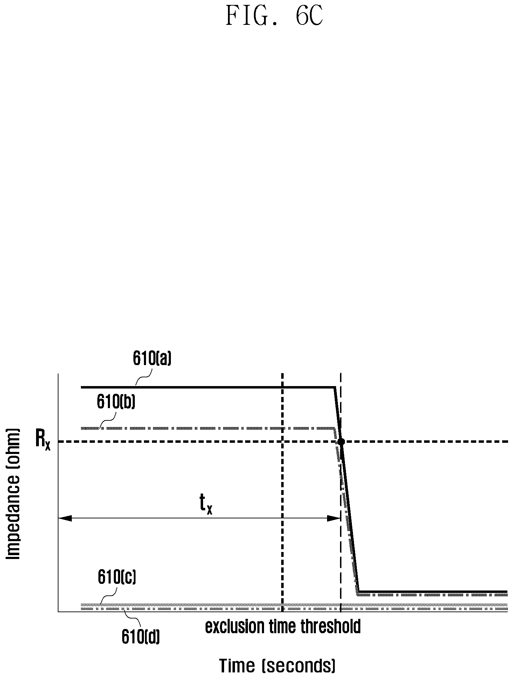

FIGS. 6A, 6B and 6C are graphs depicting contact impedance values obtained by the electronic device over time according to various embodiments.

According to various embodiments, the processor (e.g., processor 510 in FIG. 5) may perform measurement in case that all contact impedances are less than a first impedance value *Rₓ*. The processor may obtain a first contact impedance 610(a), a second contact impedance 610(b), a third contact impedance 610(c) and a fourth contact impedance 610(d) through the sensor module (e.g., sensor module 530 in FIG. 5). FIG. 6A is a graph of an embodiment in which all measured impedances decrease to less than the first impedance value *Rₓ* within an exclusion time *tₓ* that is shorter than a second time (exclusion time threshold). While the third contact impedance 610(c) and the fourth contact impedance 610(d) are less than the first impedance value *Rₓ* from the beginning, the first contact impedance 610(a) and the second contact impedance 610(b) may be greater than or equal to the first impedance value *Rₓ* when the contact impedance measurement is started. When the first contact impedance 610(a) and the second contact impedance 610(b) decrease over time to less than or equal to the first impedance value *Rₓ*, the processor may start to measure the body impedance. If the exclusion time *tₓ* during which the first contact impedance 610(a) and the second contact impedance 610(b) are greater than or equal to the first impedance value *Rₓ* is longer than the second time, the processor may determine that the body impedance measurement is a measurement failure and end the measurement.

FIGS. 6B and 6C are graphs of an embodiment in which the measurement fails because measured impedances are greater than or equal to the first impedance value *Rₓ*. With reference to FIG. 6B, while the second contact impedance 610(b) decreases to less than the first impedance value *Rₓ* within the exclusion time *tₓ* shorter than the second time, the first contact impedance 610(a) may decrease to less than the first impedance value *Rₓ* after the second time elapses. According to an embodiment, since the processor determines whether to perform the measurement based on the obtained contact impedance, it may determine whether to start the body impedance measurement based on the first contact impedance 610(a) being the highest value in the corresponding embodiment. As not all the measured impedances decrease to less than the first impedance value *Rₓ* even after the second time has elapsed, the processor may determine the measurement to be a failure. The processor may determine the embodiment of FIG. 6C as a measurement failure in the same manner. As the values of the first contact impedance 610(a) and the second contact impedance 610(b) are both greater than or equal to the first impedance value *Rₓ* during the exclusion time *tₓ* longer than the second time, the processor may determine the corresponding embodiment as a measurement failure.

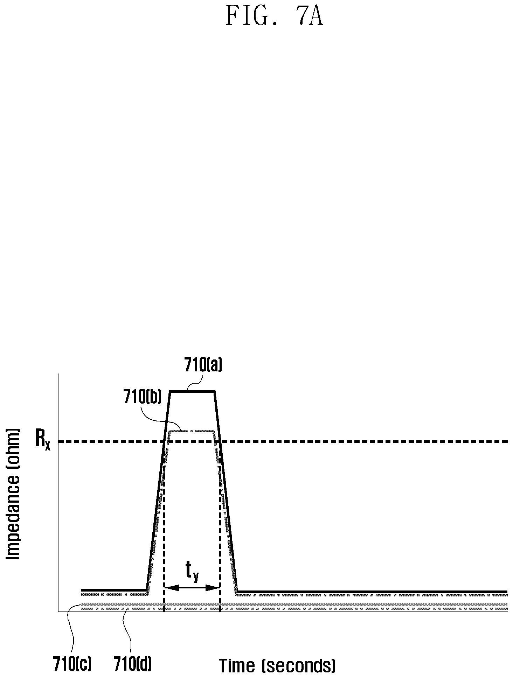

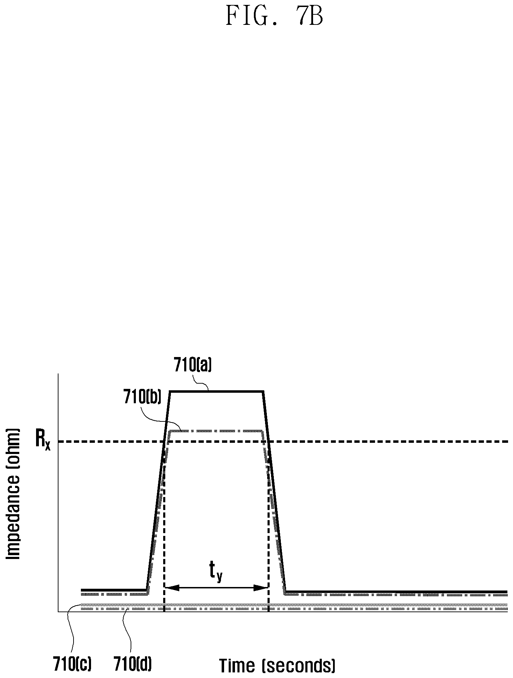

FIGS. 7A and 7B illustrate an embodiment in which an exclusion time occurs during body impedance measurement of the electronic device according to various embodiments.

According to various embodiments, the processor (e.g., processor 510 in FIG. 5) may stop the measurement when at least one contact impedance exceeds the first impedance value *Rₓ* while the body impedance measurement is in progress. FIGS. 7A and 7B show graphs of embodiments in which a stoppage has occurred during measurement. With reference to FIG. 7A, the processor may perform body impedance measurement again when all the contact impedances decrease to less than the first impedance value *Rₓ* within the exclusion time *t_{y}* shorter than the second time. The third contact impedance 710(c) and the fourth contact impedance 710(d) may be continuously measured to be less than the measurement start impedance, and the first contact impedance 710(a) and the second contact impedance 710(b) may be temporarily measured to be greater than or equal to the first impedance value *Rₓ*. The processor may stop the body impedance measurement from a time point when at least one contact impedance increases to more than the first impedance value *Rₓ*. For example, the body impedance measurement may be stopped from a time point when the first contact impedance 710(a) exceeds the first impedance value *Rₓ*. The processor may measure the exclusion time *t_{y}* from the time point when the first contact impedance 710 (a) exceeds the first impedance value *Rₓ.* Thereafter, when the contact impedance decreases again to less than the first impedance value *Rₓ*, the processor may perform the measurement again.

FIG. 7B is a graph of an embodiment in which the processor determines the measurement as a failure because the exclusion time *t_{y}* is longer than the threshold (second time). When the exclusion time *t_{y}* in which the first contact impedance 710(a) is higher than the first impedance value *Rₓ* is longer than the second time, the processor may determine the measurement as a failure.

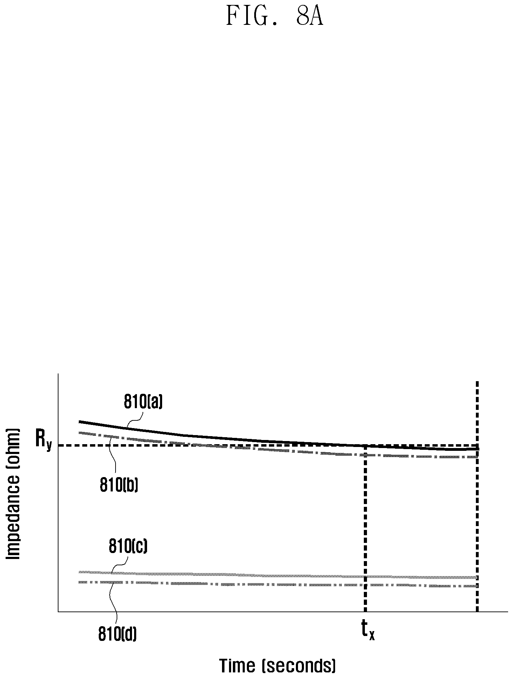

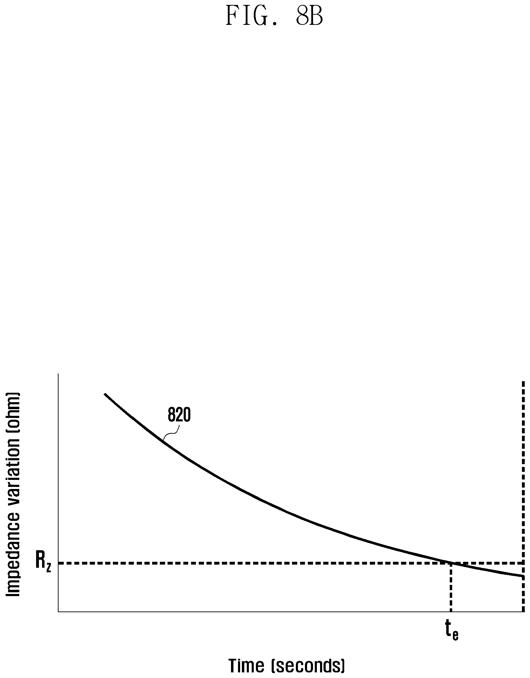

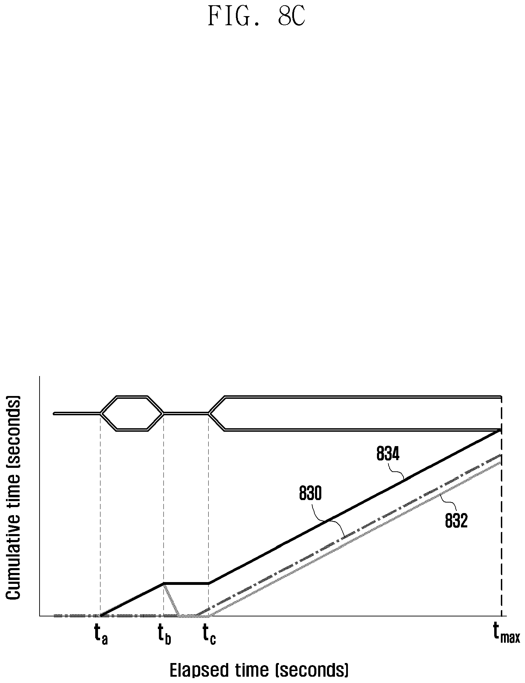

FIGS. 8A, 8B and 8C illustrate an example in which body impedance measurement of the electronic device is ended according to various embodiments.

With reference to FIG. 8A, the processor (e.g., processor 510 in FIG. 5) may end the measurement when all the contact impedances decrease to less than a second impedance value *R_{y}*. The processor may determine the second impedance value *R_{y}* at which the body impedance measurement is not affected by the contact impedance. The processor may determine the second impedance value *R_{y}* to be a value lower than the first impedance value *Rₓ* at which body impedance measurement is started. According to an embodiment, in an embodiment where whether to measure the effective time is determined using an auxiliary impedance value, the processor may set up the auxiliary impedance value to be lower than the first impedance value *Rₓ* and greater than the second impedance value *R_{y}*. The processor may obtain a first contact impedance 810(a), a second contact impedance 810(b), a third contact impedance 810(c), and a fourth contact impedance 810(d), and may end the measurement when all the contact impedances decrease to less than the second impedance value *Ry.* For example, when the largest contact impedance is the first contact impedance 810(a), the processor may end the measurement at a time point *tₓ* when the first contact impedance 810(a) decreases to less than the second impedance value *R_{y}*.

With reference to FIG. 8B, the processor may end the measurement when the fluctuation range 820 of the body impedance decreases to less than a preset value *R_{z}*. When the fluctuation range 820 of the measured body impedance is less than the preset value *R_{z}*, the processor may determine that the accuracy of the measured body impedance is sufficiently high and end the measurement. For example, the measurement may be ended at a time point *tₑ* when the fluctuation range 820 of the body impedance decreases to less than the preset value *R_{z}*.

With reference to FIG. 8C, the processor may end the measurement when the effective measurement time is longer than or equal to a first time 830. When the effective measurement time during which the body impedance is measured is longer than or equal to the first time 830, the processor may determine that the body impedance has been sufficiently accurately measured and end the measurement.

According to various embodiments, the processor may set a maximum measurement time, which is a maximum time during which body impedance measurement can be performed. The processor may perform contact impedance measurement for the maximum measurement time. According to an embodiment, the time during which the processor performs body impedance measurement based on the measured contact impedance value may not exceed the maximum measurement time. For example, the processor can treat the measurement as a success only when the measurement success condition is satisfied within the maximum measurement time. Conversely, if the body impedance measurement success condition is not satisfied for the maximum measurement time, the processor may treat this as a body impedance measurement failure.

According to various embodiments, the processor may measure the effective measurement time differently according to the size of the electrode. FIG. 8C shows graphs of an embodiment in which the measurement is started at *tₐ*, stopped at *t_{b}*, and then resumed at *t_{c}.* The first graph 832 corresponds to a case where the area of the electrode is small. In the case of a small electrode area, the processor may initialize the effective measurement time when a measurement stoppage occurs. The effective measurement time may be decreased and initialized again after *t_{b}*, and may be measured from scratch when the measurement is resumed at *t_{c}*. In this case, the effective measurement time may fail to reach the first time 830 within the maximum measurement time. The second graph 834 corresponds to a case where the area of the electrode is large. In the case of a large electrode area, the processor may not initialize the effective measurement time even when a measurement stoppage occurs. The effective measurement time measured from *tₐ* to *t_{b}* may be maintained as it is even when a measurement stoppage occurs at *t_{b}*, and then may increase cumulatively after the measurement is resumed at *t_{c}*. In this case, the effective measurement time may reach the first time 830 within the maximum measurement time; when the effective measurement time reaches the first time 830, the processor may end the measurement.

For example, consider an embodiment in which the maximum measurement time is 30 seconds, the measurement time threshold is 20 seconds, the processor performs a valid measurement during 5-7 seconds and after 12 seconds, and a measurement exclusion situation occurs during 7-12 seconds. In case that the effective measurement time is initialized because the electrode area of the electronic device is small, the processor may measure the effective measurement time again when the body impedance measurement is resumed at 12 second after measurement exclusion. Thereafter, even if the measurement is normally performed up to 30 seconds, the effective measurement time is up to 18 seconds, which cannot exceed the measurement time threshold of 20 seconds. In this case, the processor may treat it as a measurement failure. In another embodiment, when the effective measurement time is measured in a cumulative manner because the electrode area of the electronic device is large, the processor may not initialize the effective measurement time of 2 seconds measured during 5-7 seconds even if a measurement exclusion situation occurs at 7 second. Thereafter, if the measurement is normally performed up to 30 seconds, the accumulated effective measurement time becomes 20 seconds and may reach the measurement time threshold, in which case the processor may treat it as a measurement success.

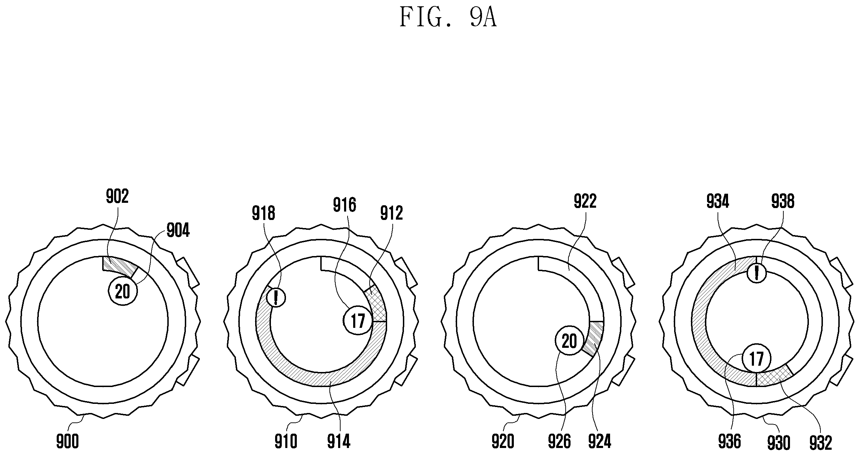

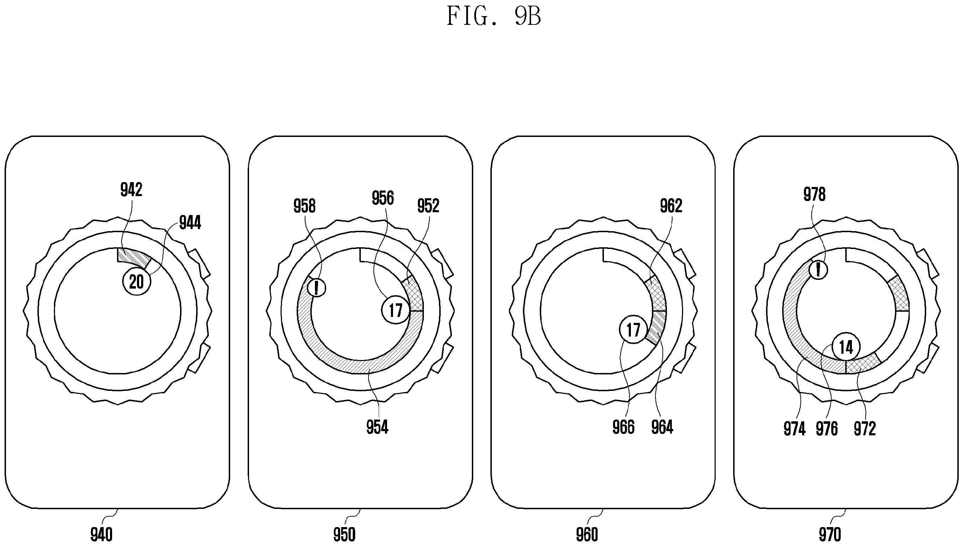

FIGS. 9A and 9B illustrate a visual interface provided by the electronic device to the user according to various embodiments.

FIG. 9A shows a visual interface provided by the processor (e.g., processor 510 in FIG. 5) when the body impedance is measured by the electronic device having a small electrode area. The processor may provide graphical bands concentric with the side bezel structure (e.g., side bezel structure 410 in FIG. 4) so that the user can identify the body impedance measurement situation at a glance. According to various embodiments, in first state 900, the processor may output, on the display (e.g., display 520 in FIG. 5), a graphical band 902 indicating the exclusion time and a graphical object 904 indicating the remaining time remaining until the end of the measurement. In first state 900, the processor has not yet started body impedance measurement, so the remaining time may be not decreased. In second state 910, the processor may start the measurement, and may output graphical bands respectively indicating the exclusion time, the effective measurement time 912, and the remaining time 914, together with a graphical object 916 indicating the remaining time and a graphical object 918 indicating the measurement end time. According to an embodiment, the processor may output the graphical bands indicating respective times in different colors so as to distinguish the exclusion time, the effective measurement time, and the remaining time from each other. For example, the exclusion time, the effective measurement time, and the remaining time may be output respectively in a first color, a second color, and a third color. As the processor is performing measurement, the remaining time may be less than that in first state 900. In third state 920, the processor may stop measuring the body impedance. Here, as the electrode area is small, the effective measurement time is initialized, and the processor may set the remaining time 926 back to the initial value. In third state 920, when the exclusion time is longer than or equal to the second time, the processor may determine this as a measurement failure. As the effective measurement time is initialized, the processor may change the graphical band 922 indicating the effective measurement time to the first color indicating the exclusion time. The processor may output a graphical band 924 indicating the exclusion time while the measurement is being stopped. In fourth state 930, the processor may resume the measurement and output the graphical bands indicating the exclusion time, the effective measurement time 932, and the remaining time 934. The processor may perform body impedance measurement in fourth state 930 as in second state 910. While the processor performs the body impedance measurement, the number input to the graphical object 936 indicating the remaining time may decrease. According to an embodiment, if the effective measurement time cannot reach the first time, the processor may output a graphical object 938 indicating the measurement end point at the start point of the graphical band.

FIG. 9B shows a visual interface provided by the processor when the body impedance is measured by the electronic device having a large electrode area. When the electrode area is large, in first state 940, the processor may output a graphical band 942 indicating the exclusion time and a graphical object 944 indicating the remaining time. In second state 950, the processor may start body impedance measurement, and may output graphical bands indicating the effective measurement time 952 and the remaining time 954, and output a graphical object 956 guiding the remaining time and a graphical object 958 guiding the end time. In third state 960, the processor may stop measuring the body impedance. Here, since the electronic device having a large electrode area does not initialize the effective measurement time, the remaining time may remain the same as in second state 950. The processor may output graphical bands indicating the effective measurement time 962 and the exclusion time 964, and may output a graphical object 966 guiding the remaining time. In fourth state 970, the processor may resume the body impedance measurement. The processor may output graphical bands indicating the exclusion time, the effective measurement time 972, and the remaining time 974, together with a graphical object 976 guiding the remaining time, and a graphical object 978 guiding the end time. In this case, since the electronic device having a large electrode area does not initialize the effective measurement time, the effective measurement time may reach the first time within the maximum measurement time.

An electronic device according to various embodiments may include: a plurality of electrodes; a sensor module operably connected to the plural electrodes; a memory; and a processor operably connected to the sensor module and the memory, wherein the processor may be configured to: obtain plural contact impedances through the sensor module based on contact between the plural electrodes and the user; perform body impedance measurement in case that all the obtained contact impedances are less than a first impedance value; and determine not to perform body impedance measurement in case that at least one of the obtained contact impedances is greater than or equal to the first impedance value.

According to various embodiments, the processor may be configured to: measure an effective measurement time being a time during which the body impedance is measured in response to determining to perform body impedance measurement; and measure an exclusion time being a time during which the body impedance is not measured in response to determining not to perform body impedance measurement.

According to various embodiments, the processor may be configured to measure the effective measurement time based on the area of the electrodes.

According to various embodiments, in case that the electrode area is less than a preset size, the processor may be configured to initialize the effective measurement time when at least one of contact impedances obtained after the starting of the body impedance measurement is greater than or equal to the first impedance value.

According to various embodiments, in case that the electrode area is greater than or equal to the preset size, the processor may be configured to measure the effective measurement time in a cumulative manner when at least one of contact impedances obtained after the starting of the body impedance measurement is greater than or equal to the first impedance value.

According to various embodiments, the processor may be configured to end the body impedance measurement in case that all the obtained contact impedances are less than a second impedance value.

According to various embodiments, the processor may be configured to: measure the fluctuation range of the obtained body impedance for a preset time; and end the measurement when the fluctuation range is less than a preset range.

According to various embodiments, the processor may be configured to end the measurement in case that the effective measurement time is longer than or equal to a preset first time.

According to various embodiments, the processor may be configured to end the measurement in case that the exclusion time is longer than or equal to a preset second time.

According to various embodiments, the processor may be configured to obtain body composition data based on the obtained body impedance.

According to various embodiments, the processor may be configured to determine the first impedance value based on the physical characteristics of the user.

According to various embodiments, the electronic device may further include a display, and the processor may be configured to output, on the display, a visual interface including graphical objects and text indicating the effective measurement time and the exclusion time.

According to various embodiments, the processor may be configured to guide the user of the effective measurement time and the exclusion time through a voice interface.

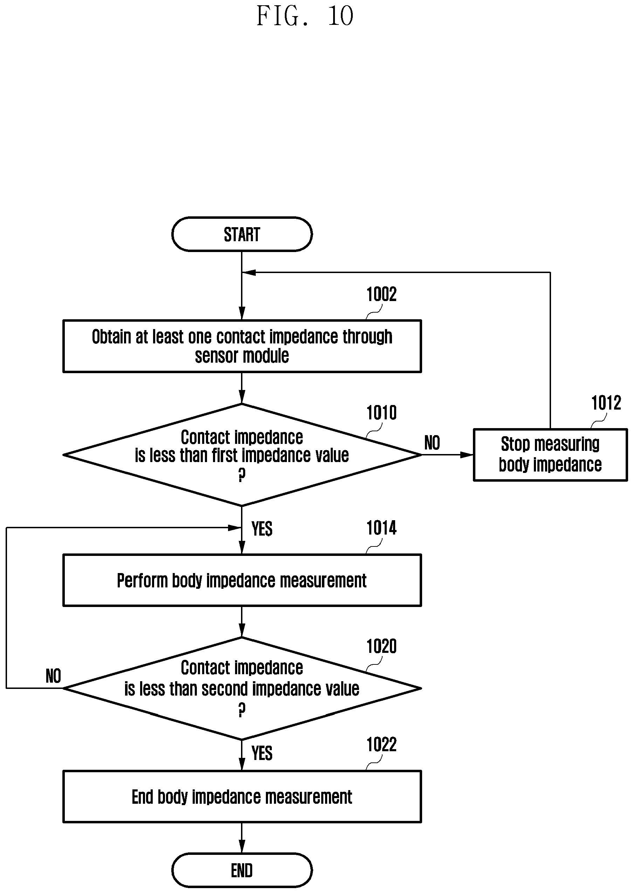

FIG. 10 is a flowchart depicting a method for the electronic device to measure body impedance according to various embodiments.

The method shown in FIG. 10 may be performed by the electronic device (e.g., electronic device 101 in FIG. 1) described with reference to FIGS. 1 to 9, and the technical features having been described above will not be described below.

According to various embodiments, the electronic device may determine the threshold value based on statistical characteristics of long-term contact impedance measurement data of the user. Also, the electronic device may determine the threshold value in further consideration of the user's body characteristics. The electronic device may obtain various body characteristics of the user by outputting an electrical signal toward the human body and obtaining impedance. The electronic device may determine a threshold value required for measuring the body impedance based on the analyzed statistical characteristics of the contact impedance measurement data and the obtained body characteristics of the user. The contact impedance is an impedance generated between the electrode of the electronic device and the user's body, and may interfere with body impedance measurement. The electronic device may set up a first impedance value, which is the upper limit of the contact impedance, in consideration of the accuracy of body impedance measurement.

In various embodiments, the electronic device may receive a threshold value through an external electronic device operably connected to the electronic device. For example, in an Internet of things (IoT) environment, the electronic device may receive a threshold value through an external electronic device connected through short-range communication (e.g., Wi-Fi or Bluetooth). The electronic device may receive a threshold value through an external electronic device connected to the same account in the server.

According to various embodiments, at operation 1002, the electronic device may obtain at least one contact impedance through the sensor module (e.g., sensor module 530 in FIG. 5). According to an embodiment, the electronic device may determine the maximum value among the obtained at least one contact impedance. The electronic device may compare a first contact impedance, a second contact impedance, a third contact impedance, and a fourth contact impedance to determine the largest value as the maximum value. According to an embodiment, the electronic device may obtain at least one contact impedance value through the sensor module at preset time intervals. Whenever it receives as many contact impedance values as the number of electrodes, the electronic device may determine the maximum value of the received values. The electrode having the maximum value may be changed according to the state of the user's body currently in contact and the value of the contact impedance measured through the sensor module.

According to various embodiments, at operation 1010, the electronic device may determine whether the contact impedance is less than the first impedance value. The electronic device may determine the first impedance value based on the user's body state, and may compare the obtained at least one contact impedance with the first impedance value. The contact impedance between the electrode and the body may decrease over time. According to an embodiment, the third and fourth contact impedances at the third and fourth electrodes in contact with the wrist may be lower than the first impedance values from the start of the measurement. In case that all the contact impedances are less than the first impedance value, the electronic device may measure the body impedance. Conversely, in case that at least one contact impedance is greater than the first impedance value, the electronic device may stop measuring the body impedance.

According to various embodiments, at operation 1012, when the body impedance measurement is stopped, the electronic device may stop measuring the effective measurement time and measure the exclusion time. The exclusion time may be a time during which the electronic device does not measure body impedance. According to an embodiment, if all contact impedances decrease to less than the first impedance value while the electronic device is measuring the exclusion time, the exclusion time may be initialized. The electronic device may measure the exclusion time from a time point when the body impedance measurement is stopped, without accumulating the exclusion time.

According to various embodiments, at operation 1014, the electronic device may measure the body impedance. The electronic device may measure the body impedance by using the sensor module.

According to various embodiments, the electronic device may measure the effective measurement time while the body impedance measurement is in progress. According to an embodiment, the electronic device may measure the effective measurement time based on the size of the area of the electrode. According to an embodiment, in case that a measurement stoppage occurs in the electronic device having an electrode area less than a preset size, the electronic device may initialize the effective measurement time. According to an embodiment, in case that a measurement stoppage occurs in the electronic device having an electrode area greater than or equal to the preset size, the electronic device may stop measuring the effective measurement time; and later when the body impedance measurement is resumed, the electronic device may measure the effective measurement time in a cumulative manner.

According to various embodiments, at operation 1020, the electronic device may determine whether the contact impedance is less than a second impedance value. The electronic device may determine the second impedance value based on the user's physical characteristics. The electronic device may perform body impedance measurement in case that the contact impedance is greater than or equal to the second impedance value, and may end the body impedance measurement in case that the contact impedance is less than the second impedance value. In case that all contact impedances are less than the second impedance value, the electronic device may determine that the contact impedance is low enough not to interfere with body impedance measurement.

According to various embodiments, at operation 1022, the electronic device may end the body impedance measurement. According to various embodiments, the electronic device may end the body impedance measurement based on at least one of the measured values. According to an embodiment, the electronic device may end the body impedance measurement in case that the fluctuation range of the measured body impedance is less than a preset range. The electronic device may measure the fluctuation range of the obtained body impedance and determine whether the fluctuation range is within the preset range. The electronic device may identify the maximum value and the minimum value among the body impedances obtained for a preset time, and calculate a difference between the maximum value and the minimum value. In case that the calculated difference between the maximum value and the minimum value is less than the preset range, the electronic device may determine that the body impedance has been stably measured and end the measurement. Conversely, when the calculated difference between the maximum value and the minimum value is greater than or equal to the preset range, the electronic device may determine that the accuracy of body impedance measurement is not sufficient and may continue to perform the measurement.

According to an embodiment, the electronic device may end the measurement in case that the effective measurement time is longer than or equal to a first time (e.g., 30 seconds). If the effective measurement time is longer than or equal to the first time, the electronic device may determine that the body impedance measurement has been sufficiently performed and may end the measurement. Even in case that at least one contact impedance is greater than or equal to the second impedance value and the body impedance variation value is greater than or equal to the preset range, if the effective measurement time is longer than or equal to the first time, the electronic device may end the measurement.

According to an embodiment, the electronic device may end the measurement when the exclusion time is longer than or equal to a second time. Unlike the aforementioned three embodiments, when the exclusion time is greater than or equal to a threshold value, the electronic device may treat this as a measurement failure. When at least one contact impedance is greater than or equal to the first impedance value, the electronic device may start to measure the exclusion time. When the time during which at least one contact impedance is greater than or equal to the first impedance value becomes longer than or equal to the second time, the electronic device may determine that the body impedance measurement is a measurement failure and may end the measurement. Similarly, even when the measurement is stopped because the contact impedance increases to more than the first impedance value while the body impedance measurement is ongoing, if the exclusion time is greater than or equal to the threshold value, the electronic device may end the measurement. According to various embodiments, the electronic device may end the body impedance measurement when any one of the above-mentioned conditions is satisfied.

According to various embodiments, after the body impedance measurement is ended, the electronic device may obtain at least one body composition data based on the obtained body impedance. The electronic device may obtain body composition data such as body fat, skeletal muscles, and body water.

According to various embodiments, the electronic device may guide the user of the measurement situation by using various interfaces. According to an embodiment, the electronic device may provide a visual interface including graphical objects and text indicating the effective measurement time and the exclusion time. According to an embodiment, the electronic device may guide the measurement situation through a voice interface.

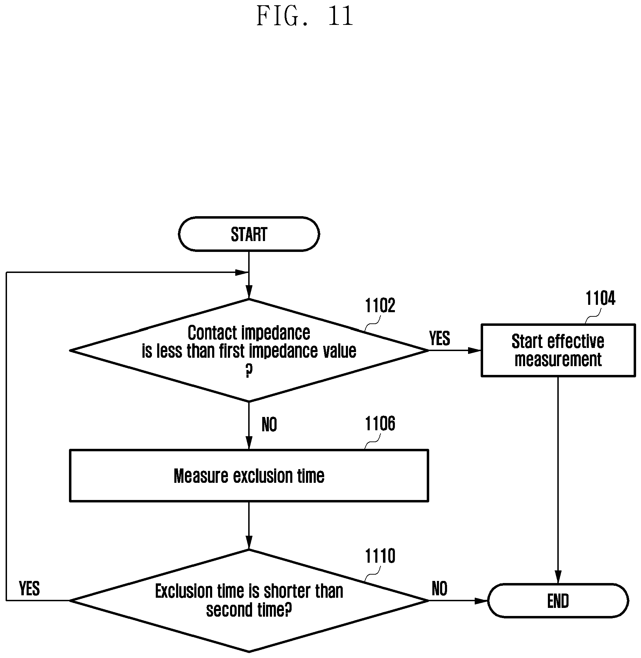

FIG. 11 is a flowchart depicting a method for the electronic device to start measurement according to various embodiments.

According to various embodiments, at operation 1102, the electronic device may determine whether the contact impedance is less than a first impedance value. The electronic device may set the first impedance value based on the user's physical characteristics. The electronic device may compare the value of at least one impedance obtained through the sensor module (e.g., sensor module 530 in FIG. 5) with the first impedance value.

According to various embodiments, at operation 1110, the electronic device may start effective measurement of the body impedance. In case that all the contact impedances are less than the first impedance value, the electronic device may start measuring the body impedance. According to an embodiment, the electronic device may measure the effective measurement time while measuring the body impedance.

According to various embodiments, at operation 1104, the electronic device may measure the exclusion time. In case that at least one contact impedance is greater than or equal to the first impedance value, the electronic device may stop measuring the body impedance. According to an embodiment, the electronic device may stop measuring the effective measurement time and measure the exclusion time.

According to various embodiments, at operation 1106, the electronic device may determine whether the exclusion time is less than a second time. In case that the exclusion time is less than the threshold, the electronic device may continue to compare the contact impedance with the first impedance value. In case that the exclusion time is greater than or equal to the threshold, the electronic device may determine the body impedance measurement to be a failure, and may end the measurement.

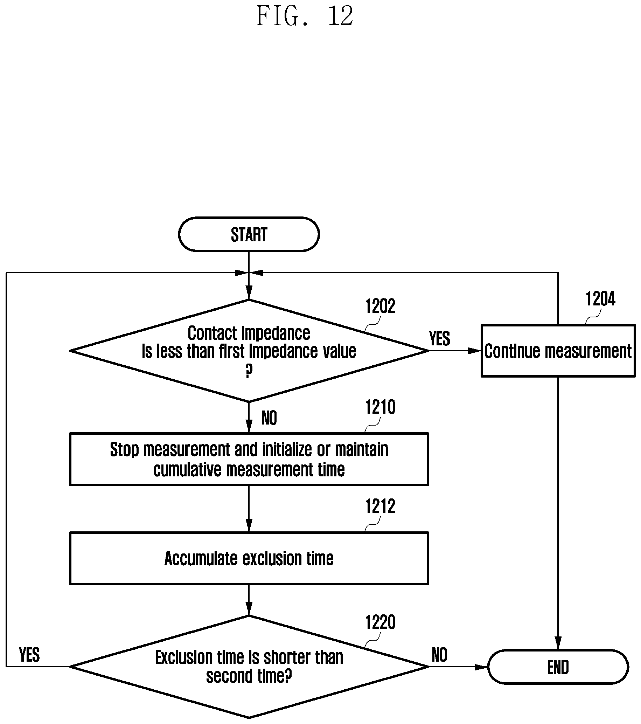

FIG. 12 is a flowchart illustrating a case in which an exclusion time occurs during measurement of the electronic device according to various embodiments.

According to various embodiments, at operation 1202, the electronic device may determine whether the contact impedance is less than a first impedance value. While measuring the body impedance, the electronic device may continuously measure the contact impedance to increase the accuracy of body impedance measurement. In case that at least one contact impedance increases to greater than or equal to the first impedance value while body impedance measurement is ongoing, the electronic device may stop measuring the body impedance.

According to various embodiments, at operation 1204, in case that all the contact impedances are less than the first impedance value, the electronic device may continue to measure the body impedance. In a state in which all the contact impedances are lower than the first impedance value, the electronic device may accurately measure the body impedance without being disturbed by the contact impedance.

According to various embodiments, at operation 1210, the electronic device may stop measuring the body impedance and initialize or maintain the effective measurement time. According to an embodiment, the electronic device may determine the effective measurement time based on the size of the electrode. In case that the area of the electrode is large, the electronic device may maintain the effective measurement time even if the measurement is stopped. Conversely, in case that the area of the electrode is small, the electronic device may initialize the effective measurement time when the measurement is stopped.

According to various embodiments, at operation 1212, the electronic device may cumulatively measure the exclusion time while the body impedance measurement is stopped. The electronic device may stop measuring the effective measurement time and measure the exclusion time while the body impedance measurement is stopped.

According to various embodiments, at operation 1220, the electronic device may determine whether the exclusion time is less than a second time. The electronic device may determine the second time in consideration of the user's physical characteristics. In case that the exclusion time is less than the threshold, the electronic device may measure the contact impedance and compare it with the first impedance value. In case that the exclusion time is greater than or equal to the threshold, the electronic device may determine the body impedance measurement to be a failure, and may end the measurement.

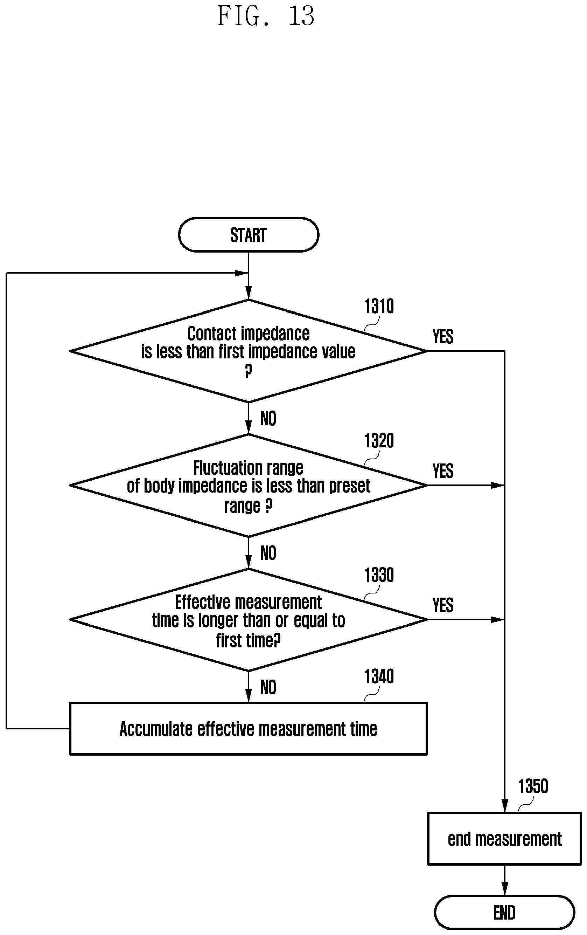

FIG. 13 is a flowchart for the electronic device to end measurement according to various embodiments.

According to various embodiments, at operation 1310, the electronic device may determine whether all contact impedances are less than a second impedance value. The electronic device may determine the second impedance value in consideration of the user's physical characteristics. According to an embodiment, the second impedance value may be determined to be lower than the first impedance value. The electronic device may end the measurement when all contact impedances are less than the second impedance value.

According to various embodiments, at operation 1320, the electronic device may determine whether the fluctuation range of the body impedance is less than a preset range. In case that the fluctuation range of the body impedance is less than the preset range, the electronic device may determine that a sufficiently accurate body impedance value has been obtained and may end the body impedance measurement. According to an embodiment, the electronic device may identify the maximum and minimum values of the body impedance for a preset time, and calculate the difference therebetween as the fluctuation range.

According to various embodiments, at operation 1330, the electronic device may determine whether the effective measurement time is longer than or equal to a first time. If the effective measurement time during which the body impedance measurement is performed is greater than or equal to the threshold value, the electronic device may determine that the body impedance measurement has been sufficiently performed and may end the measurement.

According to various embodiments, at operation 1340, the electronic device may measure the body impedance and accumulate the effective measurement time. In case that the measurement is not ended at operation 1310, 1320 or 1330, the electronic device may continuously perform the body impedance measurement. The electronic device may continuously accumulate the effective measurement time while the body impedance measurement is performed.

According to various embodiments, at operation 1350, the electronic device may end the body impedance measurement. The electronic device may obtain body composition data based on the obtained body impedance. For example, the electronic device may obtain body composition data such as body fat, skeletal muscles, and body water.

A method for an electronic device to measure body impedance according to various embodiments may include: obtaining, through a sensor module, at least one contact impedance based on contact between one of plural electrodes and the user; performing body impedance measurement when all the obtained contact impedances are less than a first impedance value; and determining not to perform body impedance measurement when at least one of the obtained contact impedances is greater than or equal to the first impedance value.

According to various embodiments, performing body impedance measurement may further include: measuring an effective measurement time being a time during which the body impedance is measured in response to determining to perform body impedance measurement; and measuring an exclusion time being a time during which the body impedance is not measured in response to determining not to perform body impedance measurement.

According to various embodiments, measuring an effective measurement time may further include measuring the effective measurement time based on the area of the electrodes.

According to various embodiments, performing body impedance measurement may further include ending the body impedance measurement in case that all the obtained contact impedances are less than a second impedance value.

According to various embodiments, performing body impedance measurement may further include: measuring the fluctuation range of the obtained body impedance for a preset time; and ending the measurement when the fluctuation range is less than a preset range.

According to various embodiments, performing body impedance measurement may further include ending the measurement in case that the effective measurement time is longer than or equal to a preset first time.

According to various embodiments, performing body impedance measurement may further include ending the measurement in case that the exclusion time is longer than or equal to a preset second time.

## Claims

1. An electronic device comprising:
a plurality of electrodes;
a sensor module operably connected to the plural electrodes;
a memory; and
a processor operably connected to the sensor module and the memory,
wherein the processor is configured to:
obtain plural contact impedances through the sensor module based on contact between the plural electrodes and a user;
perform body impedance measurement in case that all the obtained contact impedances are less than a first impedance value; and
determine not to perform body impedance measurement in case that at least one of the obtained contact impedances is greater than or equal to the first impedance value.

2. The electronic device of claim 1, wherein the processor is configured to:
measure an effective measurement time being a time during which the body impedance is measured in response to determining to perform body impedance measurement; and
measure an exclusion time being a time during which the body impedance is not measured in response to determining not to perform body impedance measurement.

3. The electronic device of claim 2, wherein the processor is configured to measure the effective measurement time based on an area of the electrodes.

4. The electronic device of claim 3, wherein, in case that the electrode area is less than a preset size, the processor is configured to initialize the effective measurement time in case that at least one of contact impedances obtained after a starting of the body impedance measurement is greater than or equal to the first impedance value.

5. The electronic device of claim 3, wherein, in case that the electrode area is greater than or equal to the preset size, the processor is configured to measure the effective measurement time in a cumulative manner in case that at least one of contact impedances obtained after a starting of the body impedance measurement is greater than or equal to the first impedance value.

6. The electronic device of claim 1, wherein the processor is configured to end the body impedance measurement in case that all the obtained contact impedances are less than a second impedance value.

7. The electronic device of claim 1, wherein the processor is configured to:
measure a fluctuation range of the obtained body impedance for a preset time; and
end the measurement in case that the fluctuation range is less than a preset range.

8. The electronic device of claim 2, wherein the processor is configured to end the measurement in case that the effective measurement time is longer than or equal to a preset first time.

9. The electronic device of claim 2, wherein the processor is configured to end the measurement in case that the exclusion time is longer than or equal to a preset second time.

10. The electronic device of claim 1, wherein the processor is configured to obtain body composition data based on the obtained body impedance.

11. The electronic device of claim 1, wherein the processor is configured to determine the first impedance value based on physical characteristics of the user.

12. The electronic device of claim 2, further comprising a display, and wherein the processor is configured to output, on the display, a visual interface including graphical objects and text indicating the effective measurement time and the exclusion time.

13. The electronic device of claim 2, wherein the processor is configured to guide the user of the effective measurement time and the exclusion time through a voice interface.

14. A method for an electronic device to measure body impedance, the method comprising:
obtaining, through a sensor module, at least one contact impedance based on contact between one of plural electrodes and a user;
performing body impedance measurement in case that all the obtained contact impedances are less than a first impedance value; and
determining not to perform body impedance measurement in case that at least one of the obtained contact impedances is greater than or equal to the first impedance value.

15. The method of claim 14, wherein performing body impedance measurement further comprises:
measuring an effective measurement time being a time during which the body impedance is measured in response to determining to perform body impedance measurement; and
measuring an exclusion time being a time during which the body impedance is not measured in response to determining not to perform body impedance measurement.
